# EUROPÄISCHE PATENTANMELDUNG

(11) **EP 2 944 324 A1**
(43) Veröffentlichungstag der Anmeldung: **18.11.2015**
(21) Anmeldenummer: 14168081.9
(22) Anmeldetag: 13.05.2014
(51) Int. Cl.: A61K 47/24, A61K 31/568, A61K 9/70

(54) **Verwendung von semifluorierten Alkanen in transdermalen therapeutischen Systemen**

(71) Anmelder: LTS LOHMANN Therapie-Systeme AG, 56626 Andernach (DE)
(72) Erfinder: Wiedersberg, Sandra, 06268 Steigra (DE)
(74) Vertreter: Schweitzer, Klaus

(57) **Zusammenfassung**

Die vorliegende Erfindung betrifft die Verwendung von semifluorierten Alkanen, insbesondere in Mischung mit Ethanol, als Lösungsvermittler, Permeationsförderer und/oder Enhancer für lipophile und/oder wenig hautpermeable pharmazeutische Wirkstoffe, die mittels eines transdermalen therapeutischen Systems (TTS) appliziert werden, und entsprechende TTS enthaltend diese Substanzen.

## Beschreibung

Die vorliegende Erfindung betrifft die Verwendung von semifluorierten Alkanen in transdermalen therapeutischen Systemen und transdermale therapeutische Systeme enthaltend semifluorierte Alkane.

Semifluorierte Alkane (SFAs) sind bekannt und auch ihre Anwendung in topisch anzuwendenden pharmazeutischen Formulierungen. Hier dienen die SFAs dazu, pharmazeutische Wirkstoffe in tiefer gelegene Hautschichten zu transportieren und dabei die äußere Hornschicht der Haut (stratum corneum = SC) zu überwinden (vgl. z.B.: EP 0670 159 A1 und WO 2012/160179 A2). Bei den Formulierungen handelt es sich meist um Lösungen oder ölige Emulsionen.

Ob die SFAs dabei in erster Linie die Funktion von Lösungsvermittlern und/oder von permeationsfördernden Stoffen übernehmen, ist noch nicht abschließend geklärt.

Diese topisch anzuwendenden pharmazeutischen Formulierungen weisen allerdings verschiedene Nachteile auf. Einmal müssen sie in der Regel mehrmals täglich angewendet werden, um die therapeutisch erforderliche Konzentration des jeweiligen Wirkstoffs am Wirkort des Patienten einzustellen. Topische Formulierungen sollen in den meisten Fällen nur eine lokale und keine systemische Wirkung erzielen. Eine zu wiederholende notwendige Anwendung führt bei allen möglichen Arzneimittelformen in der Regel zu einer unzureichenden Patienten-Compliance.

Zur systemischen Verabreichung von pharmazeutischen Wirkstoffen über die Haut sind im Stand der Technik sogenannte transdermale therapeutische Systeme (TTS) vorgeschlagen und entwickelt worden. TTS sind Darreichungsformen, die auf die Haut appliziert werden und einen Wirkstoff in die Haut abgeben, wobei dieser Wirkstoff dadurch systemisch verfügbar gemacht wird. Solche TTS, die auch als Wirkstoffpflaster bezeichnet werden, existieren in verschiedenen Ausgestaltungen und sind z.B. in WO 02/17889 A1 beschrieben. Aber auch diese Systeme sind nicht frei von Problemen in ihrer Anwendung.

Der in einem TTS enthaltene Wirkstoff muss außer der äußeren Hornschicht der Haut zunächst auch verschiedene Barrieren innerhalb des TTS, wie z.B. eine Steuermembran und die Haftklebeschicht, überwinden, wodurch letztlich der Hautflux (Permeationsrate im Fließgleichgewicht) reduziert werden kann. Bei den meisten transdermal verabreichten Wirkstoffen verläuft die Freisetzung aus dem TTS jedoch relativ schnell u. die anschließende Permeation des Wirkstoffes durch die äußerste Hornschicht, die eigentliche Permeationsbarriere der Haut, ist geschwindigkeitsbestimmend. Nur wenige Wirkstoffe, wie z.B. Nikotin permeieren die Haut so gut, dass der Wirkstoffflux mit Hilfe einer zusätzlichen Membran im TTS reduziert werden muss um die erforderlichen Blutspiegel zu erreichen.

Die meisten transdermalen Wirkstoffe permeieren die Haut jedoch eher schlecht u. den Systemen müssen noch zusätzliche Enhancer (Permeationsförderer) zugesetzt werden um die erforderlichen Blutspiegel zu erreichen.

Ein Beispiel hierfür ist die Verabreichung des lipophilen Hormons Testosteron (LogP = 3,3). In der Dissertation von Hardung (Universität Freiburg, Deutschland, 2008) wurden topische Formulierungen enthaltend Testosteron untersucht, welche SFAs als sogenannte Carrier- / oder Trägersubstanzen enthielten. Gemäß S. 103, erster vollständiger Absatz dieser Dissertation zeigen diese Formulierungen keine verbesserte Penetration für Testosteron in die unteren Schichten der Haut und damit auch keinen penetrationsverstärkenden Effekt für die Passage durch die äußere Hornhautschicht.

In der DE 10 2005 050 431 A1 werden Enhancersysteme beschrieben, welche aus mindestens 4 verschiedenen Komponenten bestehen und die Permeationsrate lipophiler und/oder wenig hautpermeabler Wirkstoffe bei Verabreichung mittels TTS erhöhen. Solche Systeme sind naturgemäß komplexer als Systeme einfacher Zusammensetzung.

Aufgabe der vorliegenden Erfindung war somit die Bereitstellung von pharmazeutischen Zubereitungen oder Mitteln, welche die für den Stand der Technik beschriebenen Nachteile nicht oder zumindest in deutlich geringerem Maß aufweisen. Weiterhin sollten entsprechende pharmazeutische Zubereitungen möglichst patientenfreundlich anwendbar und handhabbar sein.

Die erfindungsgemäße Aufgabe wird durch den Einsatz von SFAs als Lösungsvermittler und/oder permeationsfördernde Substanz und/oder als Enhancer in TTS gelöst.

Bei semifluorierten Alkanen (SFAs) handelt es sich um lineare oder verzweigte Alkane, bei denen die Wasserstoffatome teilweise durch Fluor ersetzt sind. In einer für die Zwecke der vorliegenden Erfindung bevorzugten Form bestehen diese SFAs aus einem nicht fluorierten Teil und einem perfluorierten Teil. Perfluoriert bedeutet, dass in diesem Teil des Moleküls sämtliche Wasserstoffatome durch Fluor ersetzt sind. Eine einfache Kennzeichnung solcher SFAs ist durch die Nomenklatur in Form der Abkürzungen FnHm und FnHmFo möglich. Dabei bedeuten: F = perfluoriertes Segment/Teil, H = nicht-fluoriertes Segment/Teil des SFA und n, m und o bedeuten unabhängig voneinander die Zahl der Kohlenstoffatome des jeweiligen Segments. Somit steht z.B. die Abkürzung F3H3 für 1-Perfluoropropylpropan. Da die linearen SFAs bevorzugt sind und F3H3 auch für 2-Perfluoropropylpropan, 1-Perfluoroisopropylpropan oder 2-Perfluoroisopropylpropan stehen könnte, würde dies in den letztgenannten Fällen zusätzlich gesondert vermerkt. Ohne diese Angaben stehen somit die Abkürzungen FnHm und FnHmFo für lineare SFAs. D.h., F6H8 bedeutet Perfluorohexyloctan und F4H5 Perfluorobutylpentan.

Bevorzugt bedeuten n, m, o unabhängig voneinander 3-20 Kohlenstoffatome, insbesondere weist das perfluorierte Segment des SFA 4-12 C-Atome und/oder das nichtfluorierte Segment 4-10 C-Atome auf. Bevorzugte Verbindungen sind somit F4H5, F4H6, F4H8, F6H4, F6H6, F6H8 und F6H10. Besonders bevorzugt sind F4H5, F4H6, F6H6 und F6H8 und hiervon insbesondere F6H8 und F4H5. Bezüglich der Charakterisierung der erfindungsgemäßen SFAs wird ausdrücklich auf die WO 2012/160179 A2 (Seiten 5 und 6) verwiesen.

Bei den erfindungsgemäß verwendeten SFAs kann es sich auch um Mischungen verschiedener SFA-Vertreter handeln. Bevorzugt enthalten diese wiederum zumindest eine der vorangehend genannten Substanzen.

Weiterhin wurde gefunden, dass der Zusatz von ein- und mehrwertigen Alkoholen, wie z.B. Ethanol, 1,2-Propandiol und die verschiedenen Butandiole, die lösungsvermittelnden und permeationsfördernden Eigenschaften der SFAs oder der SFA-Mischungen deutlich erhöhen kann. Bevorzugt ist hier insbesondere Ethanol, das bereits in kleinen Mengen deutliche Effekte zeigen kann. Diese sogenannte Enhancerwirkung oder Verstärkungswirkung ist für SFA/Ethanol-Mischungen besonders hoch. Bevorzugt liegt das Mischungsverhältnis von SFA(s) oder SFA-Mischungen zu Alkohol bei 1:5 bis 5:1, insbesondere bei 1:3 bis 3:1. Hierbei handelt es sich jeweils um Volumenanteile.

Die genannten Substanzen und Substanzmischungen werden erfindungsgemäß bevorzugt dazu genutzt, um insbesondere eher lipophile und/oder wenig hautpermeable Stoffe einem Patienten mittels TTS über die Haut zuzuführen.

"Lipophil" im Sinne der vorliegenden Beschreibung bedeutet, dass der Wirkstoff ein großes Bestreben hat, in eine mit Wasser nicht mischbare Phase überzugehen. Lipophile Substanzen zeichnen sich durch einen hohen LogP-Wert aus (logP > 2,5). Der LogP-Wert ist der Verteilungskoeffizient einer Substanz zwischen Octanol und Wasser: C_{Octanol}/C_{Wasser}

Unter "schlecht wasserlöslich" werden im Sinne der vorliegenden Beschreibung Wirkstoffe verstanden, die eine Löslichkeit von weniger als 0,3 Gew.-% in Wasser aufweisen, d.h. Substanzen, von denen weniger als 3 mg in einem Milliliter Wasser löslich sind.

"Wenig hautpermeabel" im Sinne der vorliegenden Beschreibung bezeichnet Wirkstoffe, deren Flux allein, d.h. ohne Permeationsverstärker oder andere Maßnahmen zur Erhöhung der Hautdurchdringung, zu gering ist, um die zur Erzielung eines therapeutischen Effekts erforderliche Wirkstoffkonzentration im Plasma erzielen zu können.

Im Allgemeinen werden Wirkstoffe, die ein Molekulargewicht >500 oder einen LogP von weniger als 1 oder mehr als 3 aufweisen, als wenig hautpermeabel angesehen.

Lipophile, schlecht wasserlösliche Wirkstoffe zeichnen sich in der Regel bei oraler Verabreichung durch eine schlechte Bioverfügbarkeit aus. Zwar ist eine transdermale Verabreichung von Wirkstoffen, die bei oraler Verabreichung schlecht bioverfügbar sind, dem Grunde nach denkbar, aber zu lipophile Wirkstoffe gehören üblicherweise auch zu den wenig bzw. schlecht hautpermeablen Wirkstoffen.

Beispiele für in diesem Sinne bevorzugte pharmazeutische lipophile Wirkstoffe sind Testosteron (LogP = 3,3), Estradiol (logP = 4.01), Buprenorphine (logP=4,98), Rotigotine (logP = 4,58), Oxybutynin (logP=4.02) u. andere Hormone.

TTS bestehen nach dem Stand der Technik aus einer arzneistoffundurchlässigen Trägerschicht (auch Rückschicht genannt), einer arzneistoffhaltigen Reservoirschicht sowie einer Haftklebeschicht zur Befestigung auf der Haut. Die letztgenannte Schicht kann auch mit der arzneistoffhaltigen Schicht identisch sein. Außerdem weisen TTS in der Regel eine vor der Applikation zu entfernende, ebenfalls wirkstoffundurchlässige Schutzschicht auf. Zusätzlich können noch andere Komponenten vorhanden sein, wie z.B. eine die Wirkstoffabgabe begrenzende Steuermembran.

Die erfindungsgemäßen TTS können sowohl in Form von Matrix-Systemen als auch in Form von Reservoir-Systemen (Reservoir-Matrix-TTS, Flüssigreservoir-Systeme) bzw. Membransystemen hergestellt werden.

Ein Matrix-System, z.B. in der Form eines monolithischen Matrix-TTS, besteht grundsätzlich aus einer schützenden Rückschicht, einer wirkstoffhaltigen Klebermatrix und einer abziehbaren Schutzfolie. Ein Reservoir-Matrix-TTS weist in der Regel eine Rückschicht, ein Flüssigkeitsreservoir, eine Steuermembran, eine Haftschicht und eine abziehbare Schutzfolie auf. Die Komponenten eines modifizierten TTS für flüchtige Wirkstoffe bestehen aus Rückschicht, Haftschicht, wirkstoffhaltigem Vlies, Haftschicht und abziehbarer Schutzfolie. Solche Systeme sind dem Fachmann bekannt und z.B. in der DE 10 2005 050 431 A1 und der WO 02/17889 A1 beschrieben.

Bevorzugt für die Zwecke der Verabreichung von leicht flüchtigen Wirkstoffen oder Zusatzstoffen (z.B. Enhancer) sind die sog. Reservoir- oder Flüssigreservoir-Systeme. Bei diesen Systemen ist ein Trocknungsprozess, bei dem der Wirkstoff oder Hilfsstoff verflüchtigt werden könnte, nicht erforderlich. Transdermale therapeutische Flüssigreservoir-Systeme an sich, bei denen eine in der Regel flüssige Wirkstoffzubereitung (als Lösung, Emulsion, Suspension) in einem aus einer die Freisetzung steuernden Membran und einer vorzugsweise wirkstoffundurchlässigen Folie gebildeten Beutel enthalten ist, sind dem Fachmann bekannt. Bei diesen Systemen dienen pharmazeutisch akzeptable und hautverträgliche organische Lösungsmittel als Trägermedium, dessen Viskosität mit geeigneten Hilfsstoffen (z.B. Mineralölen) auf die jeweiligen technologischen Erfordernisse eingestellt werden kann. Im Idealfall haben die verwendeten Lösemittel gleichzeitig die Permeation des Wirkstoffs durch die Haut des Patienten fördernde Eigenschaften. Dem Lösemittel können aber auch die Permeation des Wirkstoffs fördernde Substanzen, so genannte Enhancer, zugesetzt werden. Darüber hinaus umfassen die Flüssigreservoir-Systeme eine Haftkleberschicht, mit der das System auf der Haut des Patienten befestigt wird.

Bei dem erfindungsgemäßen Flüssigreservoir-System liegt der Wirkstoff gelöst, dispergiert, suspendiert oder emulgiert in den SFA(s), den SFA-Mischungen oder den Mischungen von einem oder mehreren SFAs mit einem oder mehreren weiteren Alkoholen vor.

Der Anteil an Wirkstoff in der wirkstoffhaltigen Zubereitung beträgt 0,1 bis 50 Gew.-%, vorzugsweise 5 bis 25 Gew.-%. Darüber hinaus kann die Wirkstoff-Zubereitung weitere Substanzen enthalten, mit denen die Abgabe des Wirkstoffs - im Sinne einer verzögerten Abgabe - gesteuert werden kann. Derartige Substanzen sind beispielsweise Absorptionsmittel.

Die Absorptionsmittel können aus der Gruppe ausgewählt sein, die Cyclodextrine, Polyvinylpyrrolidone und Cellulosederivate umfasst.

Die Wirkstoffzubereitung kann zusätzlich viskositätserhöhende Hilfsstoffe enthalten, die keine die Freisetzung steuernde Funktion haben. Vorzugsweise ist der viskositätserhöhende Hilfsstoff aus der Gruppe ausgewählt, die aus feindispersem Siliziumdioxid, beispielsweise Aerosil R 974^{®}, Polyacrylsäuren, z.B. Carbopol 934P^{®}, Mineralölen, Wollwachsen und hochmolekularen Polyethylenglykolen besteht. Ein Beispiel für ein bevorzugtes Polyethylenglykol ist Carbowax 1000^{®}. Die Wirkstoffzubereitung im Flüssigreservoir kann als Lösung, Dispersion, Suspension, Paste oder Gel vorliegen.

Bei dem erfindungsgemäßen TTS kann die Freisetzung des Wirkstoffs und der Enhancer (SFAs und deren Mischungen) aus dem Flüssig-Reservoirsystem in die Haut kontrolliert werden über:
Die Haut als Permeationsbarriere,
   - die Art der eingesetzten Steuermembran, beispielsweise durch deren chemischen Zusammensetzung und/oder der Porengröße:
   - die Art der eingesetzten Haftkleberschicht unter der Steuermembran, mit der das System auf der Haut befestigt wird, beispielswiese durch deren chemische Zusammensetzung und/oder Schichtdicke;
   - eine verzögerte Abgabe durch den Einsatz von Absorptionsmitteln im Flüssigreservoir, beispielsweise Cyclodextrinen, Polyvinylpyrrolidonen oder Cellulosederivaten.

Als Steuermembran können mikroporöse, mit definierter Porengröße hergestellte Polymerfolien aus Polypropylen, Polyurethan, Copolymeren aus Ethylen und Vinylacetat und Silikonen verwendet werden. Geeignet sind diese Polymerfolien, sofern sie beständig gegenüber den in der Wirkstoffzubereitung enthaltenen Substanzen sind.

Als Haftkleber mit die Freisetzung des pharmazeutischen Wirkstoffs steuernden Eigenschaften, die unter der Steuermembran zur Befestigung des Systems auf der Haut angebracht sind, werden Haftkleber auf Basis von Copolymeren aus Ethylen und Vinylacetat, in Kombination mit Klebharzen als Zusätze, bevorzugt. Bei diesen Haftklebern kann die Durchlässigkeit bzw. Permeabilität der Haftkleberschicht über das Verhältnis von Ethylen zu Vinylacetat eingestellt werden. Bevorzugt werden auch Haftkleber auf Basis von Silikonen, da diese für die meisten Wirk- und Hilfsstoffe permeabel sind, sowie Haftkleber auf Basis von Poly(meth)acrylaten und Haftkleber auf Basis von Polyisobutylenen.

Die vorliegende Erfindung umfasst somit
a) die Verwendung von SFAs oder Mischungen von SFAs oder Mischungen von SFAs mit Alkoholen in TTS, wobei diese als Lösungsvermittler, Permeationsförderer und/oder Enhancer wirken können,
b) TTS enthaltend die unter a) genannten Substanzen,
c) TTS enthaltend die unter a) genannten Substanzen und einen pharmazeutischen Wirkstoff, der bevorzugt lipophil ist und/oder wenig hautpermeabel ist, und
d) Die Verwendung der unter b) und c) genannten TTS zur Applikation von pharmazeutischen Wirkstoffen über die (menschliche) Haut.

Die nachfolgenden Ausführungsbeispiele dienen zur weiteren Erläuterung der Erfindung, ohne diese hierauf zu beschränken. Vielmehr sind alle genannten Merkmale in jeder für den Fachmann als geeignet erscheinenden Form frei kombinierbar, und alle diese Formen werden von der vorliegenden Erfindung umfasst.

### Beispiele 1 und 2

Für die Herstellung der haftklebenden Flüssigreservoir-Systeme wird zunächst eine wirkstofffreie Polyacrylat-Kleberlösung vom Typ Duro Tak^{®} 1050 (Fa. National Starch, Antwerpen) oder ein Copolymer aus Ethylen und Vinylacetat mit Zusatz eines Klebharzes auf Basis von Kolophonium (Foral^{®} 85 B)mit Hilfe einer Ausziehrakel in einer Nassschichtdicke von 300 µm auf einer silikonisierten Polyethylenterephthalat-Folie ausgestrichen. Anschließend werden die Lösemittel durch halbstündiges Trocknen der beschichteten Folie bei 50°C in einem Trockenschrank mit Abluftführung entfernt. Der lösemittel- und wirkstofffreie Klebefilm wird danach mit einer 35 µm dicken Polyurethanfolie (Opraflex^{®}, Fa. Lohmann) oder einer Polypropylenfolie als spätere Steuermembran durch Aufkaschieren abgedeckt. Auf die Steuermembran wird eine Polyesterfolie (Scotchpak^{®} No. 1220, Fa. 3M) aufgelegt und mit einer speziellen Siegelmaske, die über ein handelsübliches Bügeleisen aufgeheizt wird, zu Beuteln gesiegelt, die ein rundes Reservoir mit einem Durchmesser von 25 mm aufweisen.

Über eine vorhandene Öffnung zum Reservoir wird die jeweilige Mischung gemäß den Tabellen 1 und 2 mittels einer Spritze in das Reservoir gefüllt. Nach dem Befüllen des Reservoirs wird die Einfüllöffnung mit Hilfe eines Bügeleisens verschweißt, so dass ein vollständig abgeschlossenes und lagerstabiles Flüssig-Reservoirsystem entsteht.

Die Permeationsraten der vorgenannten Systeme werden am in-vitro-Diffusionsmodell der "Human-Epidermis" mit Hilfe modifizierter Franz-Diffusionszellen bestimmt. Als Akzeptormedium dient in allen Fällen Phosphatpuffer (pH 5,5) mit Zusatz von 0,1 % NaN₃ als Konservierungsmittel, thermostatiert auf 32 °C.

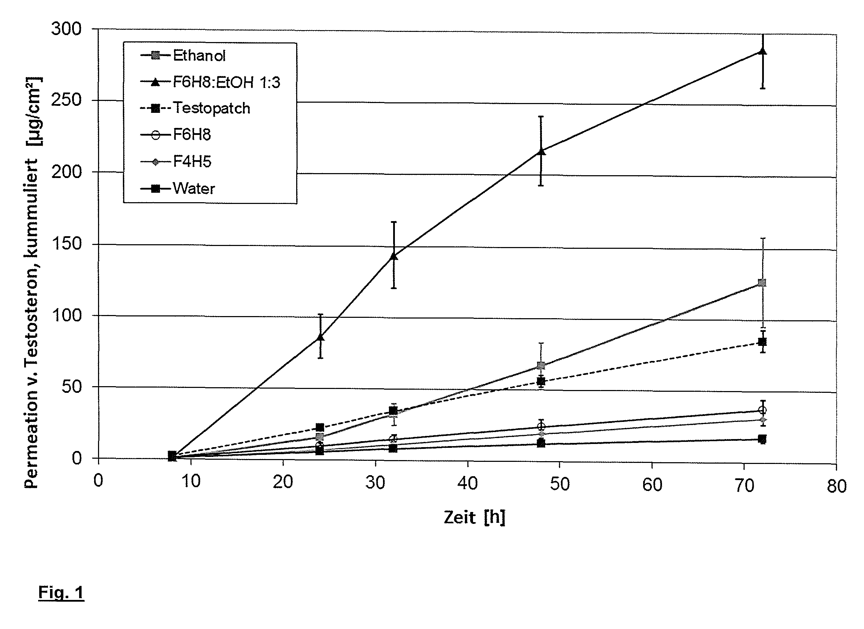

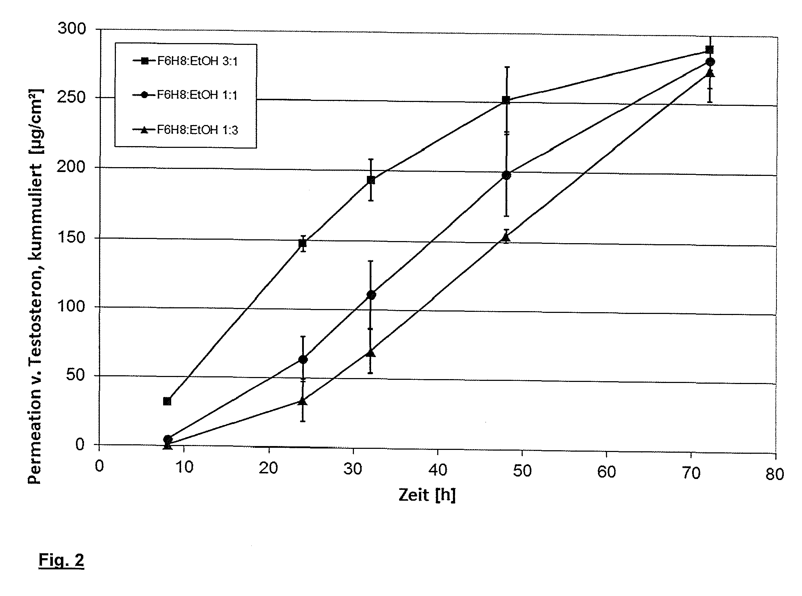

Die kumulierten Permeationsraten von Testosteron werden in den Fig. 1 und 2 dargestellt, wobei die einzelnen Messpunkte den Mittelwert aus drei Einzelmessungen darstellen. Die Ergebnisse der Untersuchungen zur Permeation von Testosteron sind in den Tabletten 1 und 2 zusammengefasst.

**Tabelle 1 (Permeation von Testosteron, gesättigte Lösung):**

| Vehikel | Sättigungslöslichkeit Cₛ [mg/ml] | Jₛₛ[µg/cm²h] | Enhance Faktor |
|---|---|---|---|
| Wasser | 0,02 | 0,2 | Referenz |
| F6H8 | 0,15 | 0,6 | 3,0 |
| F4H5 | 0,42 | 0,5 | 2,5 |
| Ethanol | 106,9 | 2,0 | 10,0 |
| Testopatch | 0,5mg/cm² | 1,3 | 6,5 |
| F6H8:Ethanol 1:3(V/V) | 80,37 | 5,5 | 27,5 |

**Tabelle 2 (Permeation von Testosteron; Lösung mit 80% der Sättigungskonzentration):**

| Vehikel | Konzentration c [mg/ml] | Jₛₛ [µg/cm²h] | Enhace Faktor |
|---|---|---|---|
| F6H8:Ethanol 3:1 (VN) | 23,1 (80% cs) | 6,8 | 34,0 |
| F6H8:Ethanol 1:1 (V/V) | 42,6 (80 % cs) | 5,6 | 28,0 |
| F6H8:Ethanol 1:3 (V/V) | 60,7 (80% cs) | 5,0 | 25,0 |

Fig. 1 zeigt die kumulierten Permeationsraten in [µg/cm²] für eine gesättigte Lösung von Testosteron in den Lösungsmitteln (Medien oder Vehikeln) gemäß der dazu korrespondierenden Tabelle 1. Die höchsten kumulierten Permeationsraten führen naturgemäß auch zum höchsten in vitro Hautflux Jₛₛ in [µg/cm² x h] gemäß Tabelle 1. Hier zeigt das System aus SFA und Alkohol (F6H8:Ethanol 1:3 (V/V) eine außerordentliche Enhancerwirkung, die auch als synergistischer Effekt bezeichnet werden kann (gegenüber Wasser liegt dieser Effekt bei einem Faktor EF=28).

Fig. 2 und die dazu korrespondierende Tabelle 2 zeigen diese Enhancerwirkung einer Mischung aus SFA und Alkohol (hier am Beispiel von F6H8 und Ethanol) für verschiedene Volumenmischungsverhältnisse (V/V) der Komponenten am Beispiel einer Testosteronlösung, welche 80% der jeweiligen Sättigungskonzentration aufweist. Die Ergebnisse zeigen eine nicht zu erwartende Enhancerwirkung (im Vergleich zu Wasser als Lösungsmittel) für alle Mischungsverhältnisse und um den Faktor EF=34 für eine Mischung aus 3 Volumenteilen F6H8 und einem Volumenteil Ethanol.

## Patentansprüche

1. Transdermales therapeutisches System (TTS) zur Applikation von pharmazeutischen Wirkstoffen über die menschliche Haut, **dadurch gekennzeichnet, dass** der oder die pharmazeutischen Wirkstoffe in Kombination mit einem oder mehreren semifluorierten Alkanen (SFAs) vorliegen.

2. TTS nach Anspruch 1, **dadurch gekennzeichnet, dass** der oder die pharmazeutischen Wirkstoffe gelöst, suspendiert oder emulgiert in einem oder in einer Mischung aus mehreren SFAs vorliegen.

3. TTS nach Anspruch 1 oder 2, **dadurch gekennzeichnet, dass** es sich bei den SFAs um lineare Alkane der allgemeinen Formel FnHm handelt, wobei F ein perfluoriertes Molekülsegment, H ein nicht-fluoriertes Molekülsegment und n und m unabhängig voneinander die Zahl der Kohlenstoffatome des jeweiligen Segments bedeuten.

4. TTS nach Anspruch 3, **dadurch gekennzeichnet, dass** n = 4-12 und m = 4-10 bedeutet.

5. TTS nach Anspruch 3 oder 4, **dadurch gekennzeichnet, dass** FnHm F4H5, F4H6, F4H8, F6H4, F6H6, F6H8 oder F6H10 bedeutet.

6. TTS nach einem der Ansprüche 1 bis 5, **dadurch gekennzeichnet, dass** das oder die SFAs in Kombination mit einem ein- oder mehrwertigen Alkohol vorliegen.

7. TTS nach Anspruch 6, **dadurch gekennzeichnet, dass** es sich um einen einwertigen Alkohol handelt.

8. TTS nach Anspruch 6 oder 7, **dadurch gekennzeichnet, dass** der Alkohol Ethanol ist.

9. TTS nach einem der Ansprüche 6 bis 8, **dadurch gekennzeichnet, dass** das Volumenverhältnis von SFA oder SFAs zu Alkohol 5:1 bis 1:5 beträgt.

10. TTS nach einem der Ansprüche 1 bis 9, **dadurch gekennzeichnet, dass** es sich bei dem Wirkstoff um einen lipophilen und/oder wenig hautpermeablen Wirkstoff handelt.

11. TTS nach einem der Ansprüche 1 bis 10, **dadurch gekennzeichnet, dass** es sich bei dem Wirkstoff um einen oder mehrere Wirkstoffe, ausgewählt aus der Gruppe: Testosteron, Estradiol, Buprenorphine, Rotigotine und andere Hormone, handelt.

12. TTS nach einem der Ansprüche 1 bis 11, **dadurch gekennzeichnet, dass** der Anteil an Wirkstoff in der wirkstoffhaltigen Zubereitung 0,1-50 Gew.-% beträgt.

13. TTS nach einem der Ansprüche 1 bis 12, **dadurch gekennzeichnet, dass** es sich bei dem TTS um ein Reservoir- oder Flüssigreservoir-System handelt.

14. Verwendung von SFA oder SFAs allein oder in Mischung mit ein- oder mehrwertigen Alkoholen als Lösungsvermittler, Permeationsförderer und/oder Enhancer für pharmazeutische Wirkstoffe in einem TTS nach einem der Ansprüche 1 bis 13.

15. Verwendung nach Anspruch 14, **dadurch gekennzeichnet, dass** eine Mischung aus einem oder mehreren SFAs mit Ethanol verwendet wird.
